# EUROPEAN PATENT APPLICATION

(11) **EP 1 515 140 A2**
(43) Date of publication of application: **16.03.2005**
(21) Application number: 04021416.5
(22) Date of filing: 09.09.2004
(51) Int. Cl.: G01N 33/487

(54) **Fluidics circuit of hermatology analyzer with heated reaction chamber**

(30) Priority: 09.09.2003 US 657948
(71) Applicant: Bayer HealthCare, LLC, Tarrytown, New York 10591 (US)
(72) Inventor: Raneri, Joseph A., West Harrison New york 10604 (US); Carlucci, Joseph, J., Harrison New York 10528 (US); Behringer, Bruce E., Park Ridge New Jersey 07656 (US); Peets, Michael T., Staatsburg New York 12580 (US); Goodsell, Terry, Danbury Connecticut 06811 (US)
(74) Representative: Ehrenstein, Wolfgang

(57) **Abstract**

The fluid circuit for a hematology analyzer includes a block-like plastic member with passageways for fluid, and a reaction chamber recess that communicates with one or more of the fluid passageways. A detachable metallic heat transfer cover member is positioned across the reaction chamber recess to close the reaction chamber recess and establish a confined reaction chamber. A heater is placed in conductive contact with the metallic heat transfer cover member to heat the cover member and thereby conduct heat to the reaction chamber. The metallic heat transfer cover member is detachably secured to the block-like plastic member and can thus be removed and replaced as needed.

## Description

### BACKGROUND OF THE INVENTION

This invention is directed to hematology analyzers incorporating a fluidics circuit with a heated reaction chamber and more particularly to a novel fluidics circuit with a replaceable metallic heat transfer member for a heated reaction chamber.

Clinical hematology analyzers are used to perform automated test analyses on body fluids, such as whole blood, to obtain data relating to the whole blood characteristics of a particular individual. The whole blood characteristics are usually compared with known acceptable whole blood parameters to determine if there are any significant deviations that might indicate a need for medical attention.

For example hematology analyses can be used to detect or confirm the existence of various illnesses, diseases and infections. Thus the hematology analysis of an individual's whole blood provides information relating to their health or medical status. Such information might dictate the need for a medical prescription, a dietary modification, an exercise program and/or other remedial measures to deal with health or medical problems that become apparent from data obtained in the hematology analysis.

One known hematology analyzer includes a fluid circuit, known as a unified fluid circuit, made of a plurality of acrylic plates that are fusion bonded, laminated or otherwise joined together to form a block-like plastic member. Some or all of the acrylic plates can be formed with an array of chamber cavity recesses pneumatic, hydraulic and vent line recesses, valve cavity recesses and reaction chamber recesses. When the acrylic plates are joined together various cavity recesses, chamber recesses and passageway recesses in the individual plates align to form a plurality of internal chambers, cavities, fluid passageways, and other internal hollow formations that constitute a network for controlling the flow and mixing of blood samples with selected fluids and reagents that are used in carrying out a hematology analysis.

U.S. Patent 5,788,927, the entire disclosure of which is incorporated by reference herein, shows a unified fluid circuit assembly for a clinical hematology instrument. The fluid circuit assembly includes a heated reaction chamber, bordered on all sides by plastic. A heating device at the reaction chamber conducts heat through a relatively thin plastic heat transfer wall of the reaction chamber, to maintain a desired temperature within the reaction chamber. The thin plastic heat transfer wall of the reaction chamber is formed as an integral part of the unified fluid circuit.

It is known that plastic, such as acrylic, is a relatively poor conductor of heat. Therefore it is necessary that the plastic heat transfer wall be as thin as possible to permit a required rate of heat transfer through the thin plastic heat transfer wall into the reaction chamber. However the relatively thin plastic heat transfer wall has been found to degrade after a period of time when subjected to heat levels that are needed to maintain the desired amount of heat transfer to the reaction chamber.

Thus, after a given amount of time, the relatively thin plastic heat transfer wall will fail. Since it is not feasible to repair a failed plastic heat transfer wall the entire unified fluid circuit must be replaced.

Because intricate molding and machining processes that entail a very high level of precision are used to manufacture the unified fluid circuit any replacement of the unified fluid circuit is extremely costly. Furthermore replacement of the unified fluid circuit also requires shutdown of the hematology analyzer. Thus the losses encountered during shutdown of the hematology analyzer for replacement of the unified fluid circuit are quite considerable. Consequently any means for prolonging of the life of the unified fluid circuit would result in substantial cost savings.

It is thus desirable to provide a unified fluid circuit having a heated reaction chamber, with a longer operating life than known unified fluidics circuits with heated reaction chambers. It is also desirable to provide a unified fluidics circuit having a heated reaction chamber with a heat transfer component that can be repaired or replaced when necessary. In addition, it is desirable to provide a uniform fluidics circuit with a heated reaction chamber that is not subjected to plastic degradation at the heat transfer surface.

### OBJECTS AND SUMMARY OF THE INVENTION

Among the several objects of the invention may be noted the provision of a novel unified fluidics circuit having a heated reaction chamber with a replaceable heat transfer member, a novel unified fluidics circuit with a replaceable heat transfer member formed of metal, a novel unified fluidics circuit with a heated reaction chamber wherein heat transfer to the reaction chamber is provided by a metal heat transfer member that is detachably secured to the unified fluid circuit and forms a structural portion of the reaction chamber, a novel unified fluidics circuit with a heated reaction chamber wherein a detachable and replaceable metallic heat transfer member forms a part of the reaction chamber and transfers heat from a heater element directly into the reaction chamber, and a novel method of transferring heat to a heated reaction chamber in a unified fluidics circuit.

Other objects and features of the invention will be in part apparent and in part pointed out hereinafter.

In accordance with the invention a unified fluidics circuit for a hematology analyzer includes a block-like plastic member formed with a plurality of interior tube-like passageways for conduction of selected fluids along selected paths defined by the passageways. One or more passageways in the block-like plastic member lead to predetermined locations where portions of the selected fluids can be mixed or reacted.

A reaction chamber recess is provided at one location in the block-like plastic member and a detachable metallic heat transfer cover member is positioned across the reaction chamber recess to close the reaction chamber recess and thereby establish a confined reaction chamber.

A heater is maintained in conductive contact with the heat transfer cover member to supply heat to the heat transfer cover member. Heat is thus conducted through the heat transfer cover member into the reaction chamber to supply heat to any contents of the reaction chamber when the metallic heat transfer cover member is in the closed position across the reaction chamber recess.

The metallic heat transfer cover member is detachably secured in the closed position at the reaction chamber recess and can be easily removed if there is a need to repair or replace either the metallic heat transfer cover member or the heater that is in contact with the heat transfer cover member.

In one embodiment of the invention the reaction chamber recess is provided at a subsurface of a vestibule formed in the block-like plastic member. The heat transfer cover member is located in the vestibule and abuts the vestibule subsurface to form a leak-tight seal at the vestibule subsurface. Preferably the heat transfer cover member includes a cover recess that aligns with the reaction chamber recess when the cover member is in the closed position.

A heater which supplies heat to the heat transfer cover member, is positioned in the vestibule in conductive contact with an outside surface of the heat transfer cover member. The heater is maintained in surface to surface contact with the heat transfer cover member. Means for maintaining the heater in conductive contact with the heat transfer cover member can include biasing means and a holding member secured to the block-like plastic member to urge the biasing means to force the heater against the outside surface of the heat transfer cover member.

The invention also includes a method of transferring heat to a reaction chamber in a fluid circuit. In accordance with the method a block-like plastic member of a fluid circuit, at one location, is formed with a reaction chamber recess. A detachable metallic heat transfer member is positioned across the reaction chamber recess to close the reaction chamber recess and establish a confined reaction chamber. The outside surface of the heat transfer member is heated with a heating device in conductive contact with the heat transfer member to transfer heat to the reaction chamber by conduction through the heat transfer member when the heat transfer member is in the closed position. In addition, the method includes detachably securing the metallic heat transfer member to the block-like plastic member in the closed position across the reaction chamber recess.

The invention accordingly comprises the constructions and methods hereinafter described, the scope of the invention being indicated in the claims.

### DESCRIPTION OF THE DRAWINGS

In the accompanying drawings,
Fig. 1 is a simplified perspective, partially exploded rear view, of a unified fluidics circuit incorporating one embodiment of the invention;
Fig. 2 is a view similar to Fig. 1, in partially assembled condition;
3 is an enlarged perspective front view of the exploded components of Fig. , minus the fasteners;
Fig. 4 is an enlarged fragmentary side sectional view of Fig. 1;
Fig. 5 is a sectional assembly view taken on the line 4-4 of Fig. 2;
Fig. 6 is a sectional assembly view taken on the line 5-5 of Fig. 2; and,
7 is a simplified schematic plan view of the unified fluidics circuit fluid flow network including various chambers, cavities, pneumatic lines, hydraulic lines and vent lines.

Corresponding reference numbers indicate corresponding parts throughout the several views of the drawings.

### DETAILED DESCRIPTION OF THE INVENTION

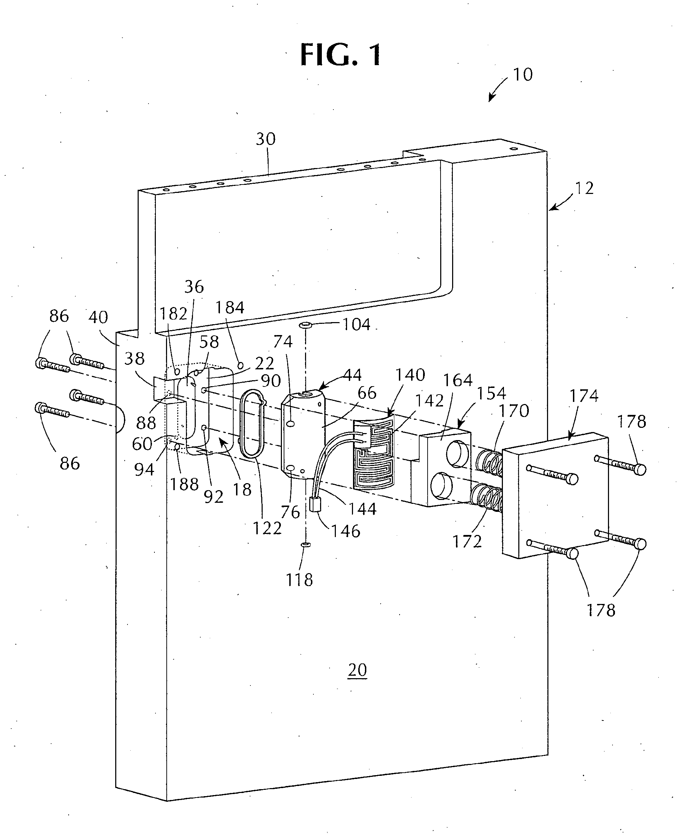

Referring to the drawings, a unified fluidics circuit of a hematology analyzer incorporating one embodiment of the invention is generally indicated by the reference number 10 in Fig. 1.

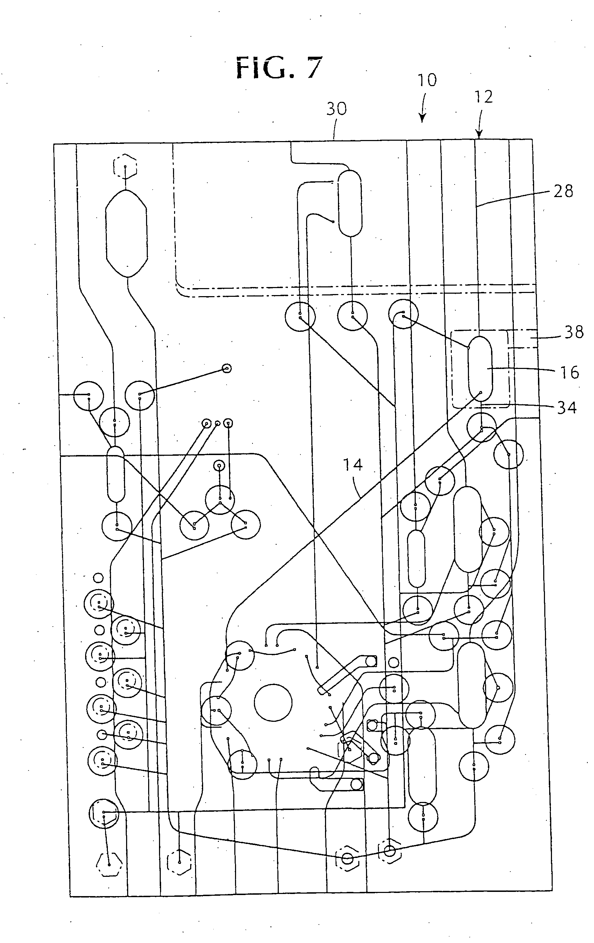

The unified fluidics circuit 10 (also referred to as the fluid circuit 10) includes a generally block-like plastic member 12 formed with a plurality of interior fluid passageways represented by circuit lines in Fig. 7. For example a fluid passageway 14 in Fig. 7 conducts fluid along a predetermined path to a reaction chamber 16 wherein fluid from the passageway 14 can be mixed and/or reacted.

The block-like plastic member 12 (also referred to as the block 12) is formed from a plurality of individual acrylic plates (not shown) that are laminated or otherwise joined together by fusion welding or other known laminating process. Each acrylic plate may include, for example, chamber recesses, valve cavity recesses and passageway recesses for pneumatic, hydraulic, and vent-lines. When the acrylic plates are laminated together, various chamber recesses and passageway recesses in the individual acrylic plates align to form a plurality of internal chambers, cavities, fluid passageways and other internal hollow formations.

The fluid circuit of Fig. 7, in the block 12, constitutes a network for controlling the flow, the mixing, and the reaction of selected fluids, including whole blood samples and reagents that are used to carry out a hematology analysis. Further details of a unified fluidics circuit for hematology analysis are disclosed in the previously referred to U.S. Patent 5,788,921 which has been incorporated by reference herein.

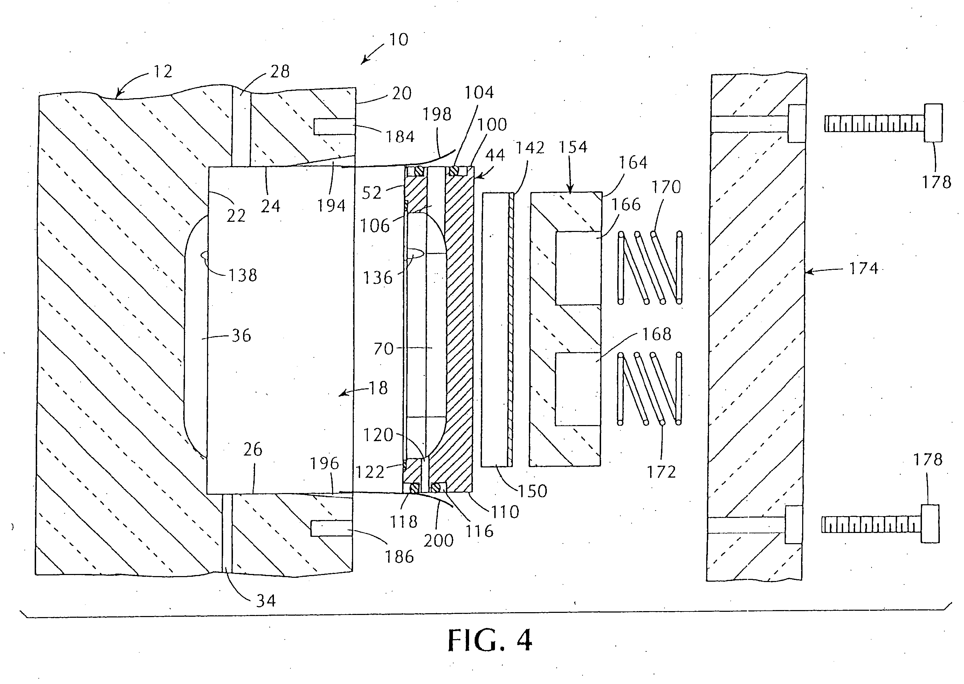

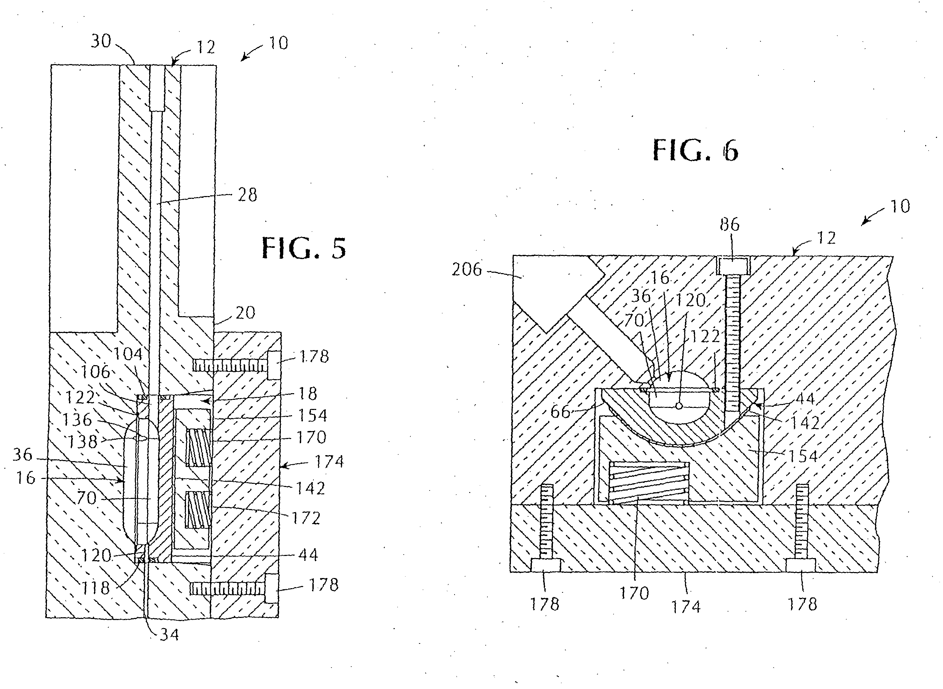

Referring to Fig. 1, the block 12 includes a vestibule 18 having a depth that extends from a surface 20 of the block 12 to a generally flat planar vestibule subsurface 22 (Fig. 4) of the block 12. The vestibule 18 also includes a top surface 24 (Fig. 4) and a bottom surface 26. A ventilation passageway 28 (Fig. 4) extends from the top surface 24 of the vestibule 18 to an upper end surface 30 (Figs. 1 and 5) of the block 12. A drain passage 34 (Figs. 4 and 5) extends from the bottom surface 26 of the vestibule 18 to a collection location (not shown).

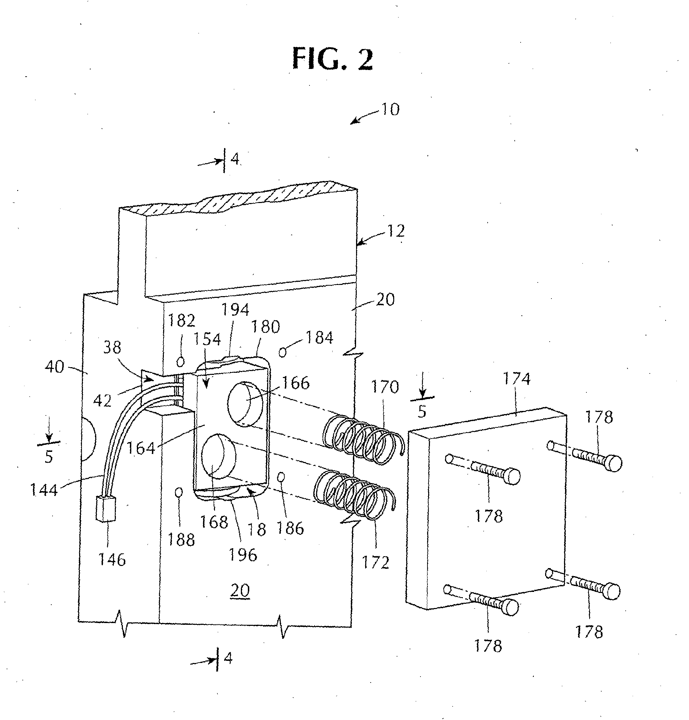

An elliptical-shaped, bulbous recess 36 (Figs. 1, 4, 5 and 6), also referred to as the reaction chamber recess 36, is formed in the vestibule subsurface 22 (Fig. 4) of the block 12. A clearance recess 38 (Figs. 1 and 2) for connector wires is formed in the'surface 20 of the block 12 and extends from the vestibule 18 to a side edge 40 of the block 12. The clearance recess 38 has a depth that extends from the block surface 20 to a subsurface 42 (Fig. 2).

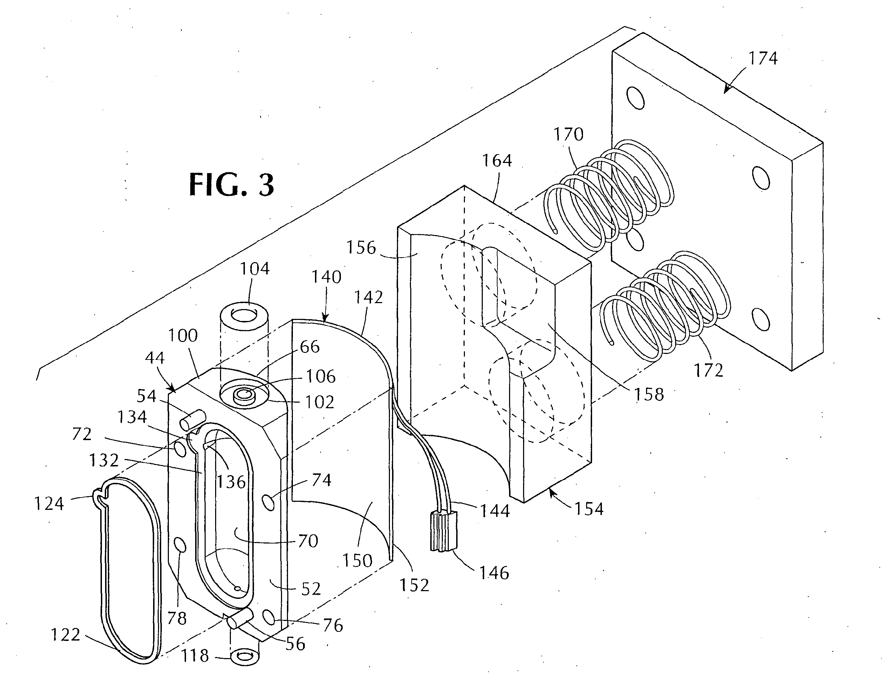

A detachable metallic heat transfer cover member 44 (most clearly shown in Figs. 1 and 3) is preferably formed of stainless steel, and is located in a closed position across the reaction chamber recess 36 as shown in Figs. 5 and 6 to cover and close the reaction chamber recess 36. The heat transfer cover member 44 (also referred to as the heat transfer member 44 or the cover member 44) includes a generally flat inside surface 52 (Figs. 3 and 4) with a pair of projecting alignment pins 54 and 56 (Fig. 3). The alignment pins 54 and 56 align with and engage corresponding holes 58 and 60 (Fig. 1) in the vestibule subsurface 22 of the block 12 when the heat transfer member 44 is in the closed position. The heat transfer member 44 also includes a curved outside surface 66 (Figs. 1 and 3) opposite the inside surface 52.

The heat transfer member 44 is formed with a cover recess 70 (Figs. 3 and 4) at the inside surface 52. The cover recess 70 aligns with the reaction chamber recess 36 when the heat transfer member 44 is in the closed position of Figs. 5 and 6. In the closed position of the heat transfer member 44 the aligned reaction chamber recess 36 and the cover recess 70 establish the confined reaction chamber 16.

The cover member 44 further includes four threaded screw holes 72, 74, 76 and 78 (Fig. 3) that extend from the inside surface 52 to the outside surface 66. The screw holes 72, 74, 76 and 78 each receive a screw 86 (Fig. 1) that passes through fastener holes 88, 90, 92 and 94 (Fig. 1) in the block 12.

A top end surface 100 (Fig. 3) of the cover member 44 is formed with an O-ring recess 102 for seating an O-ring 104. The seated O-ring 104, surrounds the port of a vent passage 106 (Fig. 4) that extends from the top end surface 100 downwardly to the cover member recess 70.

A bottom end surface 110 (Fig. 4) of the cover member 44 resembles the top end surface 100. The bottom end surface 110 is formed with an O-ring recess 116 (Fig. 4) for seating an O-ring 118 that is of smaller diameter and cross-section than the O-ring 104. The seated O-ring 118 surrounds the port of a drain passage 120 (Fig. 4) that extends upwardly from the bottom end surface 110 to the cover recess 70.

An elliptical gasket 122 (Figs. 1 and 3), with a nose portion 124 (Fig. 3), is provided between the inside surface 52 of the cover member 44 and the subsurface 22 (Fig. 1) of the vestibule 18. The gasket 122 is formed of any suitable elastomeric material such as neoprene, and seats in a gasket depression 132 (Fig. 3) at the inside surface 52 of the cover member 44, around the cover recess 70. The gasket depression 32 includes a nose section 134 (Fig. 3) that aligns with the gasket nose portion 124 and borders a rinse notch 136 (Fig. 1) at an edge of the cover recess 70. The rinse notch 136 aligns with a rinse port 138 (Figs. 4 and 5) of the reaction chamber recess 36 and communicates with a rinse passageway (not shown) in the block 2.

An electrical heater 140 (Figs. 1 and 3), in the form of a foil type resistance heater, that is well known in the art, includes a flexible heater member 42 with connector wires 144 that extend from the flexible heater member 142 to a connector 146. The flexible heater member 42 has an inside contact surface 150 (Fig. 3) that is placed against the outer surface 66 of the heat transfer cover member 44. The flexible heater member 142 also includes an outer surface 52 (Fig. 3) that is contacted by a heater engagement member 154.

The heater engagement member 54 (Fig. 3) includes a curved inner surface 156 complementary to the curved outside surface 66 of the heat transfer cover member 44 and the flexible circuit member 142 to conform the circuit member 142 against the outside surface 66 (Fig. 5) of the cover member 44. The heater engagement member 154 also includes a clearance recess 58 (Fig. 3) provided at the inner surface 156. The clearance recess 158 aligns with the clearance passage 38 (Figs. 1 and 2) in the block 12 to provide clearance for the connector wires 144 of the heater 140. The heater engagement member 154 also includes a generally planar outside surface 164 (Figs. 1, 2 and 4) having two circular spring-receiving recesses 166 and 168.

A pair of coil-shaped pressure springs 170 and 172 (Figs. 1-4), are provided in the spring recesses 166 and 168, and are held in position by a securement member 174 fastened to the block 12 by fasteners 178 (Fig. 2) that engage threaded openings 182, 184, 186 and 188 in the block surface 22.

In assembling the heat transfer cover member 44 to the block 12 the gasket 122 (Figs. 1 and 3) is disposed in the gasket depression 132 (Fig. 3) of the cover member 44. In the closed position of the cover member 44, the alignment pins 54 and 56 (Fig. 3) engage the alignment pin holes 58 and 60 (Fig. 1) in the vestibule subsurface 22. The gasket member 122 provides a leak-tight seal between the cover member 44 and the reaction chamber recess 36 (Figs 5 and 6). The reaction chamber 16 is thus established by alignment of the cover recess 70 with the reaction chamber recess 36 when the cover member 44 is in the closed position.

Placement of the cover member 44 in the closed position (Fig. 5) ensures that the vent passage 106 at the top end surface 100 of the cover member 44 and the drain passage 120 at the bottom end surface 110 of the cover member 44 respectively align with the ventilation passageway 28 and the drain passage 34 of the block 12.

The O-ring 104 provides a leak tight seal between the aligned vent passage 106 in the cover member 44 and the ventilation passageway 28 in the block 12 (Fig. 5). Similarly the O-ring 118 provides a leak tight seal between the aligned drain passage 120 in the cover member 44 and the drain passage 34 in the block 12

During installation of the cover member 44 in the vestibule 18 the O-rings 104 and 118 (Figs. 3 and 4) are seated at the respective top and bottom surfaces 100 and 110 of the cover member 44. The O-rings 104 and 118 are sized to project slightly above and slightly below the respective end surfaces 100 and 110 of the cover member 44.

Thus when the cover member 44 is being positioned in the vestibule 18 for movement toward the vestibule subsurface 22, the O-rings 104 and 118 at the top end and bottom end surfaces 100 and 10 of the cover member 44 interfere slightly with the top and bottom surfaces 24 and 26 of the vestibule 18. Also, the O-rings 104 and 118, prior to their interference entry into the vestibule 18 can respectively impinge against an upper comer edge 180 (Fig. 1) of the vestibule 18 and a lower comer edge 181 (Fig. 1) of the vestibule 18. O-ring impingement at the comer edges 180 and 181 followed by interference entry of the O-rings 104 and 118 into the vestibule 18 can damage and/or unseat the O-rings 104 and 18

To obviate impingement and interference damage to the O-rings 104 and 118 during installation of the cover member 44 into the vestibule 18 a sloping recess 194 (Fig. 4) having an approximate 6 degree incline (Figs. 3 and 4) is provided at the top surface 24 of the vestibule 18, and a corresponding sloping recess 196 (Fig. 4), having an approximate 2 degree incline, is provided at the bottom surface 26 of the vestibule 18. The sloping recesses 194 and 196 gradually compress the O-rings 104 and 118 during installation of the cover member 44.

Installation of the cover member 44 in the vestibule 18 is also facilitated by using removable mylar shims 198 and 200 (Fig. 4) at the sloping recesses 194 and 196 to shoehorn the O-rings 104 and 118 into the vestibule 18 until the cover member 44 is in a closed position, wherein the cover member gasket 122 abuts the vestibule subsurface 22. The mylar shims 198 and 200 help ensure that the O-rings 104 and 118 do not impinge against the comer edges 180 and 181 of the vestibule 18 during installation of the cover member 44.

After the cover member 44 is located in the closed position, the mylar shims 198 and 200 are pulled away from the sloping recesses 194 and 196. The fastener screws 86 (Fig. 1) are then extended through the fastener holes 88, 90, 92 and 94 (Fig. 1) for engagement with the screw holes 72, 74, 76 and 78 (Fig. 3) of the cover member 44. It should be noted that the screws 86 are sized such that they do not extend beyond the outside surface 66 of the cover member 44 (Fig. 6).

The flexible heater member 142 of the heater 140 (Figs. 1, 3 and 4) is placed on the outside surface 66 of the cover member 44 after the fasteners 86 have secured the cover member 44 to the vestibule subsurface 22. The flexible heater member 142 is positioned such that the connector wires 144 align with the wire clearance passage 38 as shown in Fig. 3.

The heater engagement member 154 is then positioned against the flexible heater member 142. Engagement between the members 142 and 154 is maintained by the pressure springs 70 and 172 and the securement member 174. which is fastened to the block surface 20 by the fasteners 178 (Fig. 5).

Under this arrangement the heater engagement member 154, the springs 70 and 72, and the securement member 174 constitute means for maintaining the heater 42 in contact with the heat transfer cover member 44.

Heat for the reaction chamber 16 is thus conducted from the flexible heater member 142 through the heat transfer cover member 44 which, as most clearly shown in Figs. 4 and 6, surrounds more then half the volume of the reaction chamber 16. Since the cover member 44 is formed of metal, the heat from the flexible heater member 142 is efficiently conducted through the cover member 44 into the reaction chamber 16.

After the cover member 44, the flexible heater member 142 and the securement member 74 are secured in position, an inlet port (not shown) can introduce whole blood and reagent into the reaction chamber 16. The whole blood and reagent are heated in the reaction chamber 16 by the heat transfer cover member 44 for a predetermined time under controlled temperature conditions that will enable the desired reaction to take place in the reaction chamber 16 between the whole blood and the reagent.

A probe 206 (Fig. 6) in the block 12 is positioned to contact the effluent medium inside the reaction chamber 16 to sense the temperature of the effluent. The probe 206 transmits an electrical signal corresponding to the sensed effluent temperature, to a feedback circuit (not shown). The feedback circuit communicates with a controller circuit (not shown) for the. heater 140 to maintain the temperature of the reaction chamber 16 at the desired temperature.

Thus if there is a deviation in the reaction chamber temperature from the desired temperature the controller circuit will cause the heater 140 to operate in a manner that raises or lowers the temperature as needed. For example, under this arrangement the temperature in the reaction chamber can be maintained at 33° C plus or minus degree.

Should there be a need to repair or replace the cover member 44, the heater 40, the heater engagement member 154, the gasket 122, the O-rings 104 and 118, the springs 170, 72 and the securement member 174, it is a relatively simple matter to remove these components from the block 12 by removing the fasteners 86 and 178.

An advantage of the invention evident from the foregoing description is a detachable metallic heat transfer member for a heated reaction chamber in a fluidics circuit. Thus the life of fluidics circuit is not limited by the life of the heat transfer structure for the heated reaction chamber since the heat transfer structure is a replaceable, detachable metallic heat transfer cover member 44.

In view of the above, it will be seen that the several objects of the invention are achieved and other advantageous results attained.

As various changes can be made in the above constructions and methods without departing from the scope of the invention, it is intended that all matter contained in the above description or shown in the accompanying drawings shall be interpreted as illustrative and not in a limiting sense.

## Claims

1. A fluid circuit for a hematology analyzer comprising,
a) a block-like plastic member formed with an interior tube-like passageway for conduction of selected fluids along a selected path defined by said passageway to a predetermined location in said block-like plastic member where portions of said selected fluids can be mixed or reacted,
b) a reaction chamber recess at said predetermined location in said block-like plastic member,
c) a detachable metallic heat transfer member positionable across said reaction chamber recess to close said reaction chamber recess and establish a confined reaction chamber,
d) a heater in conductive contact with said heat transfer member for heating said heat transfer member to transfer heat through said heat transfer member to said reaction chamber and any contents of said reaction chamber when said metallic heat transfer member is in the closed position across the reaction chamber recess, and
e) means for detachably securing said metallic heat transfer member in said closed position to said block-like plastic member at said reaction chamber recess.

2. The fluid circuit as claimed in claim 1 wherein the metallic heat transfer member has an outside metal surface in conductive contact with said heater and an opposite inside surface, and wherein the means for detachably securing said heat transfer member in said closed position include fasteners extending through aligned openings in the block-like plastic member and the heat transfer member.

3. The fluid circuit as claimed in claim 2 wherein said heat transfer member includes an inside surface recess that aligns with said reaction chamber recess when said heat transfer member is in said closed position.

4. The fluid circuit as claimed in claim 3 wherein said heat transfer member includes a top end surface and a bottom end surface, said heat transfer member further including a first vent passage portion extending from said inside surface recess through said top end surface to communicate with a second vent passage portion in said block-like plastic member, and a first drain passage portion extending from said inside surface recess through said bottom end surface to communicate with a second drain passage portion in said block-like plastic member.

5. The fluid circuit as claimed in claim 3, wherein said inside surface recess has a periphery at said inside surface of said heat transfer member and a gasket is provided at the inside surface of said heat transfer member around the periphery of said inside surface recess to provide a leak tight seal between the inside surface recess and the reaction chamber recess when the heat transfer member is in said closed position.

6. The fluid circuit as claimed in claim 1 wherein said block-like plastic member has an outside plastic surface and a vestibule is formed in the outside plastic surface, said vestibule having a depth extending from the outside plastic surface to a generally flat planar subsurface, said reaction chamber recess being formed in the subsurface of said vestibule.

7. The fluid circuit as claimed in claim 6 wherein said metallic heat transfer member has an outside metal surface in conductive contact with said heater, and an opposite inside surface formed with an inside surface recess that aligns with said reaction chamber recess when said heat transfer member is in said closed position.

8. The fluid circuit as claimed in claim 1 further including means for maintaining said heater in conductive contact with said metallic heat transfer member and wherein said metallic heat transfer member has an outside surface in conductive contact with said heater and said heater is a flexible film electric heater and said means for maintaining said heater in conductive contact with said heat transfer member includes a heater engagement member having a heat engagement surface of complementary shape with the outside surface of said heat transfer member and said flexible film electric heater when positioned against the outside surface of said heat transfer member.

9. The fluid circuit as claimed in claim 8 wherein said means for maintaining said heater in conductive contact with said heat transfer member includes means for biasing said heater engagement member against the outside metallic surface of said metallic heat transfer member to urge said heater against said outside metallic surface, said biasing means including a holding member detachably secured to the block-like plastic member and resilient members provided between the holding member and the heater to urge the heater against the outside metal surface of said metallic heat transfer member.

10. A method of transferring heat to a reaction chamber in a fluid circuit comprising,
a) forming a block-like plastic member of a fluid circuit with a reaction chamber recess at one location in the block-like plastic member,
b) positioning a detachable metallic heat transfer member across the reaction chamber recess in a closed position to close the reaction chamber recess and establish a confined reaction chamber,
c) detachably securing the metallic heat transfer member to the block-like plastic member in the closed position across the reaction chamber recess, and
d) heating an outside surface of the heat transfer member with a heating device in conductive contact with the heat transfer member to transfer heat to the reaction chamber by heat conduction through the heat transfer member when the heat transfer member is in the closed position. 868-23
